# EUROPEAN PATENT APPLICATION

(11) **EP 4 144 371 A1**
(43) Date of publication of application: **08.03.2023**
(21) Application number: 21795809.9
(22) Date of filing: 30.04.2021
(51) Int. Cl.: A61K 39/395, A61K 31/4709, A61P 35/00

(54) **COMBINATION DRUG FOR TREATING KIDNEY CANCER**

(30) Priority: 30.04.2020 CN 202010365735
(71) Applicant: Chia Tai Tianqing Pharmaceutical Group Co., Ltd., Lianyungang City, Jiangsu 222062 (CN)
(72) Inventor: YANG, Anqi, Nanjing City, Jiangsu 211100 (CN); ZHANG, Xiquan, Nanjing City, Jiangsu 211100 (CN); WANG, Xunqiang, Nanjing City, Jiangsu 211100 (CN); YU, Ding, Nanjing City, Jiangsu 211100 (CN); LI, Lin, Nanjing City, Jiangsu 211100 (CN); WANG, Rongliang, Nanjing City, Jiangsu 211100 (CN)
(74) Representative: v. Bezold & Partner Patentanwälte - PartG mbB
(86) International application number: PCT/CN2021/091592
(87) International publication number: WO 2021/219138

(57) **Abstract**

The present invention relates to the field of biomedicines. Disclosed is a combination drug for treating kidney cancer, comprising an anti-PD-L1 antibody and anlotinib or a pharmaceutically acceptable salt thereof. The present invention also provides use of the combination drug in the preparation of drugs for treating kidney cancer.

## Description

### TECHNICAL FIELD

The present invention belongs to the field of biomedicine and particularly relates to a combined pharmaceutical composition for treating kidney cancer.

### BACKGROUND

Renal cell carcinoma (RCC), also known as kidney cancer, is the most common malignancy in the urogenital system. The recent statistics in the United States show 65,340 new cases of cancer and 14,970 deaths in the United States; cancer is the leading cause of mortality. The Chinese Cancer Registry Annual Report 2018 indicates that the morbidity of kidney cancer in China was up to 40.2/100 thousand and the mortality up to 14.5/100 thousand. 74.95% of the cases were of clear cell renal cell carcinoma. Among cases of the remaining types, those of chromophobe cell carcinoma, of papillary renal cell carcinoma, of collecting duct carcinoma and of other types of cancer make up 3.21%, 2.75%, 0.56% and 18.54% of the cases, respectively.

Kidney cancer per se is not sensitive to radiotherapy and chemotherapy. Cytological therapy based on interleukin-2 (IL-2) and interferon-α (INF-α) has begun to be widely used clinically as a first-line option for adjuvant therapy since 1990. Since 2000, molecularly targeted therapy has begun to dominate the clinical treatment of kidney cancer and has been very effective in improving the prognosis of RCC. At present, small-molecule targeted drugs clinically approved by FDA mainly include sunitinib, sorafenib, pazopanib, axitinib, bevacizumab, everolimus and temsirolimus, and biological targeted drugs mainly include bevacizumab, pembrolizumab, ipilimumab, nivolumab and avelumab. Although great progress has been achieved in the treatment of kidney cancer at present, the search for other therapies is still of great clinical significance.

### SUMMARY

In one aspect, the present application provides a pharmaceutical combination or pharmaceutical composition for use in treating kidney cancer, which comprises: an anti-PD-L1 antibody, and anlotinib or a pharmaceutically acceptable salt thereof.

In another aspect, the present application provides use of a pharmaceutical combination or pharmaceutical composition of an anti-PD-L1 antibody and anlotinib or a pharmaceutically acceptable salt thereof for preparing a medicament for treating kidney cancer. Alternatively, the present application provides use of a pharmaceutical combination or pharmaceutical composition comprising an anti-PD-L1 antibody and anlotinib or a pharmaceutically acceptable salt thereof for preparing a medicament for treating kidney cancer.

In yet another aspect, the present application provides use of a pharmaceutical combination or pharmaceutical composition of an anti-PD-L1 antibody and anlotinib or a pharmaceutically acceptable salt thereof for treating kidney cancer. Alternatively, the present application provides use of a pharmaceutical combination or pharmaceutical composition comprising an anti-PD-L1 antibody and anlotinib or a pharmaceutically acceptable salt thereof for treating kidney cancer.

In yet another aspect, the present application also provides a method for treating kidney cancer, which comprises administering to a patient in need an effective amount of a pharmaceutical combination or pharmaceutical composition of an anti-PD-L1 antibody and anlotinib or a pharmaceutically acceptable salt thereof. Alternatively, the present application also provides a method for treating kidney cancer, which comprises administering to a patient in need an effective amount of a pharmaceutical combination or pharmaceutical composition comprising an anti-PD-L1 antibody and anlotinib or a pharmaceutically acceptable salt thereof.

In yet another aspect, the present application provides a kit for use in treating kidney cancer, comprising: an anti-PD-L1 antibody, and anlotinib or a pharmaceutically acceptable salt thereof, wherein the anti-PD-L1 antibody is contained in a first compartment, and anlotinib or the pharmaceutically acceptable salt thereof is contained in a second compartment; they can be administered simultaneously, separately or sequentially to a patient in need. The kit further comprises instructions for combined use of the anti-PD-L1 antibody and anlotinib or the pharmaceutically acceptable salt thereof in treating kidney cancer. In some embodiments, the kit comprises: a pharmaceutical composition of the anti-PD-L1 antibody, and a pharmaceutical composition of anlotinib or the pharmaceutically acceptable salt thereof.

In some embodiments, the pharmaceutical combination or pharmaceutical composition comprising the anti-PD-L1 antibody and anlotinib or the pharmaceutically acceptable salt thereof disclosed herein comprises: an anti-PD-L1 humanized monoclonal antibody and anlotinib or the pharmaceutically acceptable salt thereof.

In some embodiments, the pharmaceutical combination or pharmaceutical composition comprising the anti-PD-L1 antibody and anlotinib or the pharmaceutically acceptable salt thereof disclosed herein comprises: a pharmaceutical composition of the anti-PD-L1 antibody, and a pharmaceutical composition of anlotinib or the pharmaceutically acceptable salt thereof.

In some embodiments, the pharmaceutical combination or pharmaceutical composition comprising the anti-PD-L1 antibody and anlotinib or the pharmaceutically acceptable salt thereof disclosed herein comprises: a pharmaceutical composition comprising 600-2400 mg of the anti-PD-L1 antibody and a pharmaceutical composition comprising 6 mg, 8 mg, 10 mg and/or 12 mg of anlotinib or the pharmaceutically acceptable salt thereof in a single dose, wherein the pharmaceutical composition comprising the anti-PD-L1 antibody is in a single dose or in multiple doses; in some embodiments, the pharmaceutical combination or pharmaceutical composition comprises: a pharmaceutical composition comprising 600-2400 mg of the anti-PD-L1 antibody provided in multiple-dose form and a pharmaceutical composition comprising 6 mg, 8 mg, 10 mg and/or 12 mg of anlotinib or the pharmaceutically acceptable salt thereof in a single dose; in some embodiments, the pharmaceutical combination or pharmaceutical composition is a formulation suitable for administration within a single treatment cycle (e.g., a 21-day treatment cycle) and comprises a pharmaceutical composition comprising 600-2400 mg of the anti-PD-L1 antibody and a pharmaceutical composition comprising 84-168 mg of anlotinib or the pharmaceutically acceptable salt thereof.

In some embodiments, the pharmaceutical combination or pharmaceutical composition comprising the anti-PD-L1 antibody and anlotinib or the pharmaceutically acceptable salt thereof disclosed herein comprises: the anti-PD-L1 antibody and anlotinib or the pharmaceutically acceptable salt thereof in a weight ratio of (0.35-29):1, preferably (3.5-29):1, more preferably (3.5-14.5):1, and most preferably (7-14.5):1. The anti-PD-L1 antibody and anlotinib or the pharmaceutically acceptable salt thereof can be separately packaged or packaged together. Anlotinib can be packaged in multiple aliquots (e.g., 2 aliquots, 7 aliquots, 14 aliquots, 28 aliquots or more); the anti-PD-L1 antibody can be packaged in a single aliquot or in multiple aliquots (e.g., 2 aliquots, 4 aliquots or more).

In some embodiments, the present application provides a pharmaceutical combination or pharmaceutical composition comprising an anti-PD-L1 antibody and anlotinib or a pharmaceutically acceptable salt thereof, wherein the anti-PD-L1 antibody and anlotinib or the pharmaceutically acceptable salt thereof are each in the form of a pharmaceutical composition and can be administered simultaneously, sequentially or at intervals. Still further, the anti-PD-L1 antibody is administered once every week, every 2 weeks, every 3 weeks, or every 4 weeks; preferably, the anti-PD-L1 antibody is administered at a dose of 600-2400 mg each time. Still further, anlotinib or the pharmaceutically acceptable salt thereof is administered at a dose of 6 mg, 8 mg, 10 mg or 12 mg once daily on a regimen of consecutively 2-week treatment and then 1-week interruption.

In some embodiments, the pharmaceutical combination or pharmaceutical composition comprising the anti-PD-L1 antibody and anlotinib or the pharmaceutically acceptable salt thereof disclosed herein comprises a pharmaceutical composition of the anti-PD-L1 antibody and a pharmaceutical composition of anlotinib, wherein the anti-PD-L1 antibody is prepared in a single dose or in multiple doses suitable for providing 600-2400 mg of the anti-PD-L1 antibody for a patient at first administration, and anlotinib or the pharmaceutically acceptable salt thereof is prepared in single doses suitable for providing 6 mg, 8 mg, 10 mg and/or 12 mg of anlotinib or the pharmaceutically acceptable salt thereof for a patient daily for 14 consecutive days.

In some embodiments, the pharmaceutical combination or pharmaceutical composition comprising the anti-PD-L1 antibody and anlotinib or the pharmaceutically acceptable salt thereof disclosed herein comprises: a pharmaceutical composition of the anti-PD-L1 antibody in which the anti-PD-L1 antibody is at a concentration of 10-60 mg/mL, and a pharmaceutical composition comprising 6 mg, 8 mg, 10 mg and/or 12 mg of anlotinib or the pharmaceutically acceptable salt thereof in a single dose.

In some embodiments, the pharmaceutical combination or pharmaceutical composition comprising the anti-PD-L1 antibody and anlotinib or the pharmaceutically acceptable salt thereof disclosed herein comprises: a pharmaceutical composition of the anti-PD-L1 antibody in which the anti-PD-L1 antibody is at a concentration of 10 mg/mL, and a pharmaceutical composition comprising 8 mg, 10 mg and/or 12 mg of anlotinib or the pharmaceutically acceptable salt thereof in a single dose.

In some embodiments, the pharmaceutical combination or pharmaceutical composition comprising the anti-PD-L1 antibody and anlotinib or the pharmaceutically acceptable salt thereof disclosed herein comprises: a pharmaceutical composition comprising 1200 mg of the anti-PD-L1 antibody provided in multiple-dose form, and a pharmaceutical composition comprising 8 mg, 10 mg and/or 12 mg of anlotinib or the pharmaceutically acceptable salt thereof in a single dose.

In some embodiments, the kit is suitable for administration within a single treatment cycle (e.g., a 21-day treatment cycle) and comprises a pharmaceutical composition comprising 600-2400 mg of the anti-PD-L1 antibody and a pharmaceutical composition comprising 84-168 mg of anlotinib or the pharmaceutically acceptable salt thereof.

### Anlotinib or pharmaceutically acceptable salt thereof

The chemical name of anlotinib is 1-[[[4-(4-fluoro-2-methyl-1*H*-indol-5-yl)oxy-6-methoxyquinolin-7-yl]oxy]methyl]cyclopropylamine, which has the following structural formula:

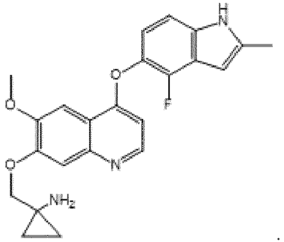

The pharmaceutically acceptable salts of anlotinib include, but are not limited to, salts formed from anlotinib and acids selected from the group consisting of the following: hydrochloric acid, hydrobromic acid, sulfuric acid, nitric acid, phosphoric acid, acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, p-toluenesulfonic acid, 3-phenylpropionic acid, trimethylacetic acid, t-butylacetic acid, dodecyl sulfuric acid, gluconic acid, glutamic acid, hydroxynaphthoic acid, salicylic acid and stearic acid; in some embodiments, the pharmaceutically acceptable salt is a hydrochloride or a maleate; in some embodiments, the pharmaceutically acceptable salt is a dihydrochloride.

The dose of anlotinib or the pharmaceutically acceptable salt thereof referred to in the present application is based on the molecular weight of the free base of anlotinib, unless otherwise stated.

Anlotinib or the pharmaceutically acceptable salt thereof can be administered through various routes including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular and intrathecal administrations. In some specific embodiments, the drug is administered orally. The amount of anlotinib or the pharmaceutically acceptable salt thereof administered can be determined according to the severity of the disease, the response of the disease, any treatment-related toxicity and the age and health of a patient. For example, the daily dose of anlotinib or the pharmaceutically acceptable salt thereof can be 2 mg to 20 mg. In some embodiments, the daily dose of anlotinib or the pharmaceutically acceptable salt thereof can be 2 mg, 3 mg, 4 mg, 5 mg, 6 mg, 7 mg, 8 mg, 9 mg, 10 mg, 11 mg, 12 mg, 13 mg, 14 mg, 15 mg and 16 mg. Anlotinib or the pharmaceutically acceptable salt thereof can be administered once or multiple times daily. In some embodiments, anlotinib or the pharmaceutically acceptable salt thereof is administered once daily in the form of a solid oral formulation.

The administration regimen of anlotinib or the pharmaceutically acceptable salt thereof can be determined depending on the combination of the activity and toxicity of the drug, tolerance of a patient, etc. Preferably, anlotinib or the pharmaceutically acceptable salt thereof is administered at intervals. The interval administration includes a treatment period and an interruption period. Anlotinib or the pharmaceutically acceptable salt thereof can be administered once or multiple times daily in the treatment period. In some embodiments, the ratio of the treatment period to the interruption period in days is 2:(0.5-5), preferably 2:(0.5-3), more preferably 2:(0.5-2), and even more preferably 2:(0.5-1). In some embodiments, anlotinib or the pharmaceutically acceptable salt thereof is administered on a regimen of consecutively 2-week treatment and then 2-week interruption. In some embodiments, anlotinib or the pharmaceutically acceptable salt thereof is administered on a regimen of consecutively 2-week treatment and then 1-week interruption. In some embodiments, anlotinib or the pharmaceutically acceptable salt thereof is administered on a regimen of consecutively 5-day treatment and then 2-day interruption. For example, anlotinib or the pharmaceutically acceptable salt thereof can be orally administered at a dose of 6 mg, 8 mg, 10 mg or 12 mg once daily on a regimen of consecutively 2-week treatment and then 1-week interruption.

### Pharmaceutical composition of anlotinib or pharmaceutically acceptable salt thereof

In some embodiments of the present application, a single dose of the pharmaceutical composition of anlotinib or the pharmaceutically acceptable salt thereof comprises 6 mg, 8 mg, 10 mg or 12 mg of anlotinib.

In some embodiments of the present application, according to a treatment cycle of 2-week treatment and then 1-week interruption, a total dose of the pharmaceutical composition of anlotinib or the pharmaceutically acceptable salt thereof administered per cycle is 84-168 mg. In some embodiments, a total dose of the pharmaceutical composition of anlotinib or the pharmaceutically acceptable salt thereof includes an amount selected from the group consisting of 84 mg, 112 mg, 140 mg and 168 mg or from a range formed of any of the aforementioned values. In some embodiments, a total dose of the pharmaceutical composition of anlotinib or the pharmaceutically acceptable salt thereof is preferably 112-168 mg.

In some embodiments, the pharmaceutical composition is an oral formulation; in some embodiments, the pharmaceutical composition is an oral solid formulation; in some embodiments, the pharmaceutical composition includes, but is not limited to, a tablet, and a capsule.

### Anti-PD-L1 antibody

In some embodiments of the present application, the anti-PD-L1 antibody is the antibody disclosed in WO2016022630 or CN107001463A.

In some embodiments of the present application, the anti-PD-L1 antibody comprises the following amino acid sequences: a heavy chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 1 or SEQ ID NO: 4; a heavy chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 2 or SEQ ID NO: 5; a heavy chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 3 or SEQ ID NO: 6; a light chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 7 or SEQ ID NO: 10; a light chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 8 or SEQ ID NO: 11; and a light chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 9 or SEQ ID NO: 12.

In some embodiments of the present application, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

In some embodiments of the present application, an isolated anti-PD-L1 antibody described herein comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

Each of the CDR regions described herein and the variants thereof described above are capable of specifically recognizing and binding to PD-L1, thereby effectively blocking the signaling between PD-L1 and PD-1.

In some embodiments of the present application, the anti-PD-L1 antibody comprises the following amino acid sequences: a heavy chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 13 or SEQ ID NO: 14; and a light chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 15 or SEQ ID NO: 16.

In some embodiments of the present application, the anti-PD-L1 antibody comprises the following amino acid sequences: a heavy chain variable region set forth in SEQ ID NO: 13, and a light chain variable region set forth in SEQ ID NO: 15.

In some embodiments of the present application, the anti-PD-L1 antibody comprises the following amino acid sequences: a heavy chain variable region set forth in SEQ ID NO: 14, and a light chain variable region set forth in SEQ ID NO: 16.

In some embodiments of the present application, the anti-PD-L1 antibody comprises the following amino acid sequences: a heavy chain amino acid sequence set forth in SEQ ID NO: 17, and a light chain amino acid sequence set forth in SEQ ID NO: 18.

In some embodiments of the present application, the anti-PD-L1 antibody comprises the following amino acid sequences: a heavy chain amino acid sequence set forth in SEQ ID NO: 19, and a light chain amino acid sequence set forth in SEQ ID NO: 20.

In some embodiments of the present application, the anti-PD-L1 antibody comprises the following amino acid sequences: a heavy chain amino acid sequence set forth in SEQ ID NO: 21, and a light chain amino acid sequence set forth in SEQ ID NO: 18.

In one specific embodiment, the anti-PD-L1 humanized mAb disclosed herein comprises one or more conservatively substituted variants selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21. The humanized anti-PD-L1 mAb comprising the conservatively substituted variants retains the ability to specifically recognize and bind to PD-L1.

In some embodiments of the present application, the anti-PD-L1 antibody may be an IgG1 or IgG4 antibody.

In some embodiments of the present application, the anti-PD-L1 antibody is an IgG1 antibody. In some embodiments, the anti-PD-L1 antibody is a glycosylated IgG1 antibody.

In some embodiments of the present application, the anti-PD-L1 antibody comprises heavy chain complementarity determining regions (CDRs) selected from the group consisting of heavy chain CDRs derived from antibodies 13C5 and 5G11, and light chain CDRs selected from the group consisting of light chain CDRs derived from antibodies 13C5 and 5G11. In one embodiment, the anti-PD-L1 antibody disclosed herein comprises: a heavy chain variable region selected from the group consisting of heavy chain variable regions of chimeric antibodies ch5G11-hIgG1, ch5G11-hIgG4, ch13C5-hIgG1 and ch13C5-hIgG4; and a light chain variable region selected from the group consisting of light chain variable regions of chimeric antibodies ch5G11-hIgG1, ch5G11-hIgG4, ch13C5-hIgG1 and ch13C5-hIgG4. In one embodiment, the anti-PD-L1 antibody disclosed herein comprises: a heavy chain variable region selected from the group consisting of heavy chain variable regions of humanized antibodies hu13C5-hIgG1, hu13C5-hIgG4, hu5G11-hIgG1 and hu5G11-hIgG4; and a light chain variable region selected from the group consisting of light chain variable regions of humanized antibodies hu13 C5-hIgG 1, hu13C5-hIgG4, hu5G11-hIgG1 and hu5G11-hIgG4. Reference can be made to the description of the patents WO2016022630 or CN107001463A: 13C5, ch13C5-hIgG1, ch13C5-hIgG4, hu13C5-hIgG1 or hu13C5-hIgG4 comprises an HCDR1 sequence of SYGMS (SEQ ID NO: 4), an HCDR2 sequence of SISSGGSTYYPDSVKG (SEQ ID NO: 5), an HCDR3 sequence of GYDSGFAY (SEQ ID NO: 6), an LCDR1 sequence of ASQSVSTSSSSFMH (SEQ ID NO: 10), an LCDR2 sequence of YASNLES (SEQ ID NO: 11), and an LCDR3 sequence of QHSWEIPYT (SEQ ID NO: 12); 5G11, ch5G11-hIgG1, ch5G11-hIgG4, hu5G11-hIgG1 or hu5G11-hIgG4 comprises an HCDR1 sequence of TYGVH (SEQ ID NO: 1), an HCDR2 sequence of VIWRGVTTDYNAAFMS (SEQ ID NO: 2), an HCDR3 sequence of LGFYAMDY (SEQ ID NO: 3), an LCDR1 sequence of KASQSVSNDVA (SEQ ID NO: 7), an LCDR2 sequence of YAANRYT (SEQ ID NO: 8), and an LCDR3 sequence of QQDYTSPYT (SEQ ID NO: 9).

In some embodiments of the present application, the anti-PD-L1 antibody in the pharmaceutical combination may be selected from one or more. As used herein, the term "more" refers to more than one, for example, two, three, four, five or more. For example, in some embodiments of the present application, the anti-PD-L1 antibody is selected from the group consisting of an antibody comprising a heavy chain variable region set forth in SEQ ID NO: 13 and a light chain variable region set forth in SEQ ID NO: 15, or selected from the group consisting of an antibody comprising a heavy chain variable region set forth in SEQ ID NO: 14 and a light chain variable region set forth in SEQ ID NO: 16, or selected from the group consisting of a combination thereof. As another example, the anti-PD-L1 antibody is selected from the group consisting of an antibody comprising a heavy chain amino acid sequence set forth in SEQ ID NO: 17 and a light chain amino acid sequence set forth in SEQ ID NO: 18, or selected from the group consisting of an antibody comprising a heavy chain amino acid sequence set forth in SEQ ID NO: 19 and a light chain amino acid sequence set forth in SEQ ID NO: 20, or selected from the group consisting of an antibody comprising a heavy chain amino acid sequence set forth in SEQ ID NO: 21 and a light chain amino acid sequence set forth in SEQ ID NO: 18, or selected from the group consisting of combinations of any of the foregoing.

In some embodiments, the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

### Pharmaceutical composition of anti-PD-L1 antibody

In some embodiments of the present application, a single dose of the pharmaceutical composition of the anti-PD-L1 antibody comprises 300 mg or 600 mg of the anti-PD-L1 antibody.

In some embodiments of the present application, a total dose of the pharmaceutical composition of the anti-PD-L1 antibody is 600-2400 mg. In some embodiments, a total dose of the pharmaceutical composition of the anti-PD-L1 antibody includes an amount selected from the group consisting of 600 mg, 900 mg, 1200 mg, 1500 mg, 1800 mg, 2100 mg and 2400 mg or from a range formed of any of the aforementioned values. In some embodiments, a total dose of the pharmaceutical composition of the anti-PD-L1 antibody is preferably 600-2100 mg or 900-1500 mg.

In some embodiments of the present application, the pharmaceutical composition of the anti-PD-L1 antibody is a solution for injection. In some embodiments of the present application, the pharmaceutical composition of the anti-PD-L1 antibody comprises one or more of a buffer, an isotonicity modifier, a stabilizer and/or a surfactant. In particular, the pharmaceutical composition of the anti-PD-L1 antibody comprises 1-150 mg/mL anti-PD-L1 antibody (e.g., mAb), 3-50 mM buffer, 2-150 mg/mL isotonicity modifier/stabilizer and 0.01-0.8 mg/mL surfactant, and has a pH of about 4.5-6.8.

In some embodiments of the present application, in the pharmaceutical composition of the anti-PD-L1 antibody, the anti-PD-L1 mAb is at a concentration of about 5-150 mg/mL, preferably about 10-60 mg/mL, and more preferably about 10-30 mg/mL (w/v). In some specific embodiments, the anti-PD-L1 mAb is at a concentration of about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 110 mg/mL, or about 120 mg/mL; preferably about 10 mg/mL, about 20 mg/mL, about 30 mg/mL, about 40 mg/mL, about 50 mg/mL, or about 60 mg/mL; more preferably about 10 mg/mL, about 20 mg/mL, or about 30 mg/mL (w/v). In some embodiments, the anti-PD-L1 mAb is at a concentration of about 10 mg/mL (w/v). In other embodiments, the anti-PD-L1 mAb is at a concentration of about 30 mg/mL (w/v). In other embodiments, the anti-PD-L1 mAb is at a concentration of about 60 mg/mL (w/v).

In some embodiments of the present application, the buffer is a histidine salt buffer. The histidine salt buffer is at a concentration of about 5-30 mM, preferably about 10-25 mM, more preferably about 10-20 mM, and most preferably about 10-15 mM. In some specific embodiments, the histidine salt buffer is at a concentration of about 5 mM, about 10 mM, about 15 mM, about 20 mM, about 25 mM, or about 30 mM. In some embodiments, the histidine salt buffer is at a concentration of about 10 mM. In other embodiments, the histidine salt buffer is at a concentration of about 15 mM. In other embodiments, the histidine salt buffer is at a concentration of about 20 mM. The histidine salt buffer comprises histidine and hydrochloric acid.

In some embodiments of the present application, the isotonicity modifier/stabilizer is sucrose at about 20-150 mg/mL, preferably sucrose at about 40-100 mg/mL, and more preferably sucrose at about 60-80 mg/mL (w/v). In some specific embodiments, the sucrose is at a concentration of about 40 mg/mL, 50 mg/mL, 60 mg/mL, 70 mg/mL, 80 mg/mL, 90 mg/mL or 100 mg/mL. In some specific embodiments, the sucrose is at a concentration of about 60 mg/mL. In some specific embodiments, the sucrose is at a concentration of about 70 mg/mL. In some specific embodiments, the sucrose is at a concentration of about 80 mg/mL. In some specific embodiments, the sucrose is at a concentration of about 90 mg/mL.

In some embodiments of the present application, the surfactant is selected from the group consisting of polysorbate 80, polysorbate 20 and poloxamer 188; preferably polysorbate 80 and polysorbate 20; and more preferably polysorbate 80. In some embodiments, the surfactant is at a concentration of about 0.05-0.6 mg/mL, preferably about 0.1-0.4 mg/mL, and more preferably about 0.2-0.3 mg/mL (w/v).

In some embodiments of the present application, the surfactant is polysorbate 80 or polysorbate 20 at about 0.01-0.8 mg/mL (w/v). In some specific embodiments, the surfactant is polysorbate 80 at about 0.05-0.6 mg/mL, preferably polysorbate 80 at about 0.1-0.4 mg/mL, more preferably polysorbate 80 at about 0.2-0.3 mg/mL, and most preferably polysorbate 80 at about 0.2 mg/mL. In some embodiments, the pharmaceutical composition has a polysorbate 80 content of about 0.1 mg/mL, 0.2 mg/mL, 0.3 mg/mL, 0.4 mg/mL, 0.5 mg/mL, or 0.6 mg/mL; preferably, the pharmaceutical composition has a polysorbate 80 content of about 0.2 mg/mL, 0.3 mg/mL, 0.4 mg/mL, or 0.5 mg/mL; more preferably, the pharmaceutical composition has a polysorbate 80 content of about 0.2 mg/mL, 0.3 mg/mL, or 0.4 mg/mL; most preferably, the pharmaceutical composition has a polysorbate 80 content of about 0.2 mg/mL. In some embodiments, the pharmaceutical composition has a polysorbate 80 content of about 0.1 mg/mL. In other embodiments, the pharmaceutical composition has a polysorbate 80 content of about 0.2 mg/mL. In some embodiments, the pharmaceutical composition has a polysorbate 80 content of about 0.3 mg/mL. In other embodiments, the pharmaceutical composition has a polysorbate 80 content of about 0.4 mg/mL. In some embodiments, the pharmaceutical composition has a polysorbate 80 content of about 0.5 mg/mL.

In some embodiments of the present application, an aqueous solution of the pharmaceutical composition has a pH selected from 4.0-6.8, preferably 4.5-6.5, more preferably 5.5-6.0, and most preferably 5.5. In some embodiments, an aqueous solution of the pharmaceutical composition has a pH of about 4.5, about 4.8, about 5.0, about 5.2, about 5.4, about 5.5, about 5.6, about 5.8 or about 6.0, preferably about 5.0, about 5.2, about 5.4, about 5.5 or about 5.6, and more preferably about 5.5. In some embodiments, an aqueous solution of the pharmaceutical composition has a pH of about 5.0. In some embodiments, an aqueous solution of the pharmaceutical composition has a pH of about 5.2. In some embodiments, an aqueous solution of the pharmaceutical composition has a pH of about 5.4. In some embodiments, an aqueous solution of the pharmaceutical composition has a pH of about 5.5. In some embodiments, an aqueous solution of the pharmaceutical composition has a pH of about 5.6. In some embodiments, an aqueous solution of the pharmaceutical composition has a pH of about 5.8. In some embodiments, an aqueous solution of the pharmaceutical composition has a pH of about 6.0.

In some specific embodiments of the present application, the pharmaceutical composition comprises: (a) an anti-PD-L1 antibody at a concentration of about 20 mg/mL (w/v), (b) sucrose at a concentration of about 70 mg/mL (w/v), (c) polysorbate 80 at a concentration of about 0.1 mg/mL (w/v), (d) histidine at a concentration of about 20 mM, and (e) optionally a suitable amount of hydrochloric acid for adjusting the pH of the composition to about 5.0. In one specific embodiment of the present application, the pharmaceutical composition comprises: (a) an anti-PD-L1 mAb at a concentration of about 20 mg/mL (w/v), (b) sucrose at a concentration of about 70 mg/mL (w/v), (c) polysorbate 80 at a concentration of about 0.1 mg/mL (w/v), (d) histidine at a concentration of about 20 mM, and (e) optionally a suitable amount of hydrochloric acid for adjusting the pH of the composition to about 5.0.

In another specific embodiment of the present application, the pharmaceutical composition comprises: (a) an anti-PD-L1 antibody at a concentration of about 10 mg/mL (w/v), (b) sucrose at a concentration of about 80 mg/mL (w/v), (c) polysorbate 80 at a concentration of about 0.2 mg/mL (w/v), (d) histidine at a concentration of about 10 mM, and (e) optionally a suitable amount of hydrochloric acid for adjusting the pH of the composition to about 5.5.

In another specific embodiment of the present application, the pharmaceutical composition comprises: (a) an anti-PD-L1 antibody at a concentration of about 50 mg/mL (w/v), (b) sucrose at a concentration of about 80 mg/mL (w/v), (c) polysorbate 80 at a concentration of about 0.3 mg/mL (w/v), (d) histidine at a concentration of about 10 mM, and (e) optionally a suitable amount of hydrochloric acid for adjusting the pH of the composition to about 5.5.

In another specific embodiment of the present application, the pharmaceutical composition comprises: (a) an anti-PD-L1 antibody at a concentration of about 100 mg/mL (w/v), (b) sucrose at a concentration of about 80 mg/mL (w/v), (c) polysorbate 80 at a concentration of about 0.5 mg/mL (w/v), (d) histidine at a concentration of about 10 mM, and (e) optionally a suitable amount of hydrochloric acid for adjusting the pH of the composition to about 5.5.

In another specific embodiment of the present application, the pharmaceutical composition comprises: (a) an anti-PD-L1 antibody at a concentration of about 30 mg/mL (w/v), (b) sucrose at a concentration of about 80 mg/mL (w/v), (c) polysorbate 80 at a concentration of about 0.2 mg/mL (w/v), (d) histidine at a concentration of about 10 mM, and (e) optionally a suitable amount of hydrochloric acid for adjusting the pH of the composition to about 5.5.

In another specific embodiment of the present application, the pharmaceutical composition comprises: (a) an anti-PD-L1 antibody at a concentration of about 60 mg/mL (w/v), (b) sucrose at a concentration of about 80 mg/mL (w/v), (c) polysorbate 80 at a concentration of about 0.2 mg/mL (w/v), (d) histidine at a concentration of about 10 mM, and (e) optionally a suitable amount of hydrochloric acid for adjusting the pH of the composition to about 5.5.

In another specific embodiment of the present application, the pharmaceutical composition comprises: (a) an anti-PD-L1 antibody at a concentration of about 10 mg/mL (w/v), (b) sucrose at a concentration of about 70 mg/mL (w/v), (c) polysorbate 80 at a concentration of about 0.4 mg/mL (w/v), (d) histidine at a concentration of about 20 mM, and (e) optionally a suitable amount of acetic acid for adjusting the pH of the composition to about 6.5.

In another specific embodiment of the present application, the pharmaceutical composition comprises: (a) an anti-PD-L1 mAb at a concentration of about 10 mg/mL (w/v), (b) sucrose at a concentration of about 80 mg/mL (w/v), (c) polysorbate 80 at a concentration of about 0.2 mg/mL (w/v), (d) histidine at a concentration of about 20 mM, and (e) optionally a suitable amount of hydrochloric acid for adjusting the pH of the composition to about 5.5.

In another specific embodiment of the present application, the pharmaceutical composition is a water-soluble injection, including but not limited to a water-soluble formulation without lyophilization or a water-soluble formulation reconstituted from a lyophilized powder. In other embodiments, the pharmaceutical composition is a lyophilized formulation. The lyophilized formulation refers to a formulation prepared by subjecting an aqueous solution to a lyophilization process in which a substance is first frozen, and then the amount of a solvent is reduced by sublimation (primary drying process) and then by desorption (secondary drying process) until the amount of the solvent is reduced to a value that no longer supports a biological activity or a chemical reaction. The lyophilized formulations of the present application can also be dried by other methods known in the art, such as spray drying and bubble drying.

### Kidney cancer

In the present application, the kidney cancer includes hereditary kidney cancer and sporadic kidney cancer.

In some embodiments of the present application, the kidney cancer includes clear cell renal cell carcinoma and non-clear cell renal cell carcinoma.

In the present application, the kidney cancer includes, but is not limited to, clear cell renal cell carcinoma, multilocular cystic renal neoplasm of low malignant potential, papillary renal cell carcinoma, hereditary leiomyomatosis disease and renal cell carcinoma syndrome-associated renal cell carcinoma, chromophobe renal cell carcinoma, collecting duct carcinoma, renal medullary carcinoma, MiT family translocation renal cell carcinoma, succinate dehydrogenase-deficient renal carcinoma, mucinous tubular and spindle cell carcinoma, tubulocystic renal cell carcinoma, acquired cystic disease associated renal cell carcinoma, clear cell papillary renal cell carcinoma, unclassified renal cell carcinomas, papillary adenoma, and oncocytoma, based on histological typing.

In some embodiments of the present application, the kidney cancer is selected from clear cell renal cell carcinoma, or from a renal cell carcinoma dominated by clear cell components.

In some embodiments of the present application, a patient with the kidney cancer is a low-risk kidney cancer patient, a medium-risk kidney cancer patient, or a high-risk kidney cancer patient. In some embodiments, a patient with the kidney cancer is a low-risk renal cell carcinoma patient, or a medium-risk renal cell carcinoma patient. In some embodiments, a patient with the kidney cancer is an advanced medium-risk renal cell carcinoma patient.

In some embodiments of the present application, the kidney cancer includes, but is not limited to, localized kidney cancer, locally advanced kidney cancer, synchronous metastatic kidney cancer, and metachronous metastatic kidney cancer. In some embodiments, the kidney cancer is locally advanced kidney cancer. In other embodiments, the kidney cancer is metastatic kidney cancer.

In some embodiments of the present application, the kidney cancer is treatment-naive renal cell carcinoma. In some embodiments of the present application, the kidney cancer is treatment-naive advanced renal cell carcinoma. In some embodiments, the kidney cancer is treatment-naive advanced clear cell renal cell carcinoma or treatment-naive advanced non-clear cell renal cell carcinoma. In some embodiments, the kidney cancer is treatment-naive advanced clear cell renal cell carcinoma or treatment-naive advanced renal cell carcinoma dominated by clear cell components.

In some embodiments of the present application, the kidney cancer is one that has previously been treated with one or more treatment regimens that ended in failure; in some embodiments of the present application, the kidney cancer is one that has previously been treated with one, two, three, four, or five treatment regimens that ended in failure.

In some embodiments of the present application, the kidney cancer is selected from recurrent or refractory kidney cancer; in some embodiments of the present application, a patient with the kidney cancer suffers a relapse after being treated with a prior treatment regimen and achieving objective response, or a patient with the kidney cancer does not achieve objective response to a prior treatment regimen.

In some embodiments of the present application, the kidney cancer is one that has not been subjected to systemic prior treatment. In some embodiments of the present application, the kidney cancer is one that has previously been treated with cytokine therapy that ended in failure and/or is drug resistant.

In the present application, the prior treatment includes, but is not limited to, surgical treatment, medical treatment, and/or interventional treatment.

In some embodiments of the present application, the prior treatment includes, but is not limited to, surgical treatment, radiotherapy, and/or medication. The surgical treatment includes, but is not limited to, nephron sparing surgery, radical nephrectomy, lymph node dissection, inferior vena cava tumor thrombosis surgery, tumor-reducing nephrectomy, and oligometastasis resection. The radiotherapy includes, but is not limited to, symptom-reducing radiotherapy or radical radiotherapy. The medication includes, but is not limited to, chemotherapy, cytokine therapy, and/or targeted drug therapy.

In some embodiments of the present application, the medication includes, but is not limited to, administration of one or more of cyclophosphamide, ifosfamide, vincristine, prednisone, prednisolone, methylprednisolone, fluorouracils (including but not limited to 5-fluorouracil, tegafur-gimeracil-oteracil potassium, tegafur, and capecitabine), bicins (including but not limited to adriamycin, pirarubicin, amrubicin, epirubicin, aclarubicin, and idarubicin), antifolates (including but not limited to methotrexate), cytarabine, azacitidine, fludarabine, bendamustine, mitoxantrone, etoposide, procarbazine, gemcitabine, mesna, dexamethasone, leucovorin, platins (including but not limited to oxaliplatin, cisplatin, carboplatin, nedaplatin, dicycloplatin, and miriplatin), camptothecin analogs (including but not limited to camptothecin, hydroxycamptothecin, irinotecan, and topotecan), taxanes (including but not limited to paclitaxel, albumin-bound paclitaxel and docetaxel), IL-2, INF-α, sunitinib, sorafenib, pazopanib, axitinib, lenvatinib, cabozantinib, everolimus, temsirolimus, tivozanib, bevacizumab, pembrolizumab, ipilimumab, nivolumab and avelumab.

In some embodiments of the present application, the medication is a combination of a small-molecule targeted drug and a biological targeted drug, including but not limited to the combination of avelumab and axitinib, the combination of nivolumab and ipilimumab, the combination of pembrolizumab and axitinib, or the combination of atezolizumab and bevacizumab.

In yet another aspect, the present application also provides an anti-PD-L1 antibody for use in treating kidney cancer. The present application also provides a method for treating kidney cancer, which comprises administering to a patient in need an effective amount of the anti-PD-L1 antibody of the present application. The present application also provides use of the anti-PD-L1 antibody for treating kidney cancer. The present application also provides use of the anti-PD-L1 antibody for preparing a medicament for treating kidney cancer.

### Administration regimen of pharmaceutical combination or pharmaceutical composition

In some embodiments of the present application, in the use or treatment method described above, the anti-PD-L1 antibody and anlotinib or the pharmaceutically acceptable salt thereof are each in the form of a pharmaceutical composition and can be administered simultaneously, sequentially or at intervals.

In some embodiments of the present application, in the use or treatment method described above, the anti-PD-L1 antibody and anlotinib are separately administered at intervals. In some embodiments, the antibody and anlotinib are administered separately on the same regimen or different regimens. In some embodiments, the two are separately administered on different regimens.

In some embodiments of the present application, in the use or treatment method described above, the anti-PD-L1 antibody can be administered once every week (q1w), once every 2 weeks (q2w), once every 3 weeks (q3w), or once every 4 weeks (q4w). In one specific embodiment, the anti-PD-L1 antibody is administered once every 3 weeks. In some embodiments, the anti-PD-L1 antibody is administered at a dose of 600-2400 mg each time.

Anlotinib or the pharmaceutically acceptable salt thereof can be administered at a dose of 6 mg, 8 mg, 10 mg or 12 mg once daily on a regimen of consecutively 2-week treatment and then 1-week interruption.

In some embodiments, the anti-PD-L1 antibody and anlotinib or the pharmaceutically acceptable salt thereof are used in identical or different treatment cycles. In some specific embodiments, the anti-PD-L1 antibody and anlotinib are used in identical treatment cycles, e.g., in 1-week, 2-week, 3-week or 4-week treatment cycles.

In some embodiments of the present application, in the use or treatment method, one treatment cycle comprises 21 days; the PD-L1 antibody is administered on the first day of each treatment cycle, and anlotinib or the pharmaceutically acceptable salt thereof is administered daily on days 1-14 of each cycle. In one specific embodiment, the PD-L1 antibody is administered once on the first day of each treatment cycle, and anlotinib or the pharmaceutically acceptable salt thereof is administered once daily on days 1-14 of each cycle.

In some embodiments of the present application, in the use or treatment method, the anti-PD-L1 antibody can be administered to a subject at a dose selected from the group consisting of 0.01 to 40 mg/kg, 0.1 to 30 mg/kg, 0.1 to 20 mg/kg, 0.1 to 15 mg/kg, 0.1 to 10 mg/kg, 1 to 15 mg/kg, 1 to 20 mg/kg, 1 to 3 mg/kg, 3 to 10 mg/kg, 3 to 15 mg/kg, 3 to 20 mg/kg, 3 to 30 mg/kg, 10 to 20 mg/kg, or 15 to 20 mg/kg, or administered to a subject at a dose of 60 mg to 2400 mg, 90 mg to about 1800 mg, 120 mg to 1500 mg, 300 mg to 900 mg, 600 mg to 900 mg, 300 mg to 1200 mg, 600 mg to 1200 mg, or 900 mg to 1200 mg.

In some embodiments for the use or treatment method, one treatment cycle comprises 21 days; 1200 mg of the PD-L1 antibody is administered on the first day of each treatment cycle, and 6 mg, 8 mg, 10 mg and/or 12 mg of anlotinib or the pharmaceutically acceptable salt thereof is administered daily on days 1-14 of each cycle.

In some embodiments of the present application, in a 3-week treatment cycle, the anti-PD-L1 antibody and anlotinib or the pharmaceutically acceptable salt thereof are administered to a subject in a weight ratio of (0.35-29):1, preferably (3.5-29):1, more preferably (3.5-14.5):1, and most preferably (7-14.5):1, wherein the anti-PD-L1 antibody and anlotinib or the pharmaceutically acceptable salt thereof are administered in a single dose and multiple doses, respectively.

### Administration

The content below is not intended to limit the administration of the pharmaceutical combination disclosed herein.

The components in the pharmaceutical combination or pharmaceutical composition of the present application can be administered independently, or some or all of the components are co-administered through various proper routes, including, but not limited to, oral administration or parenteral administration (through intravenous, intramuscular, local or subcutaneous routes). In some embodiments, the components in the pharmaceutical combination disclosed herein can be administered independently, or some or all of the components are co-administered by means of oral administration or injection, for example, intravenous injection or intraperitoneal injection.

The components in the pharmaceutical composition disclosed herein can be formulated independently in suitable dosage forms, or some or all of the components are co-formulated in a suitable dosage form including, but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant and dosage forms of sustained-released formulations for oral or non-oral administration.

The components in the pharmaceutical combination disclosed herein can be formulated independently, or some or all of the components are co-formulated with a pharmaceutically acceptable carrier and/or excipient.

The pharmaceutical combination disclosed herein may further comprise an additional therapeutic agent. In one embodiment, the additional therapeutic agent can be a kidney cancer therapeutic agent known in the art.

The pharmaceutical combination or pharmaceutical composition of the present application described above has the following beneficial effects:
(1) better efficacy in controlling tumor growth or even eliminating tumors as compared with either drug of the combination administered alone;
(2) fewer doses as compared with either drug of the combination administered alone;
(3) good tolerability in patients, and fewer adverse effects and/or complications as compared with either drug administered alone;
(4) a higher disease control rate in patients treated;
(5) longer survivals (e.g., median survival, progression-free survival, or overall survival) in patients treated;
(6) longer survivals (e.g., median survival, progression-free survival, or overall survival) in patients treated as compared with standard chemotherapies;
(7) a longer duration of response (DOR); and/or
(8) better activity in treating kidney cancer and better anti-tumor synergistic effect, as compared with either drug of the combination administered alone.

### Definitions and description

Unless otherwise stated, the following terms used herein shall have the following meanings. A certain term, unless otherwise specifically defined, should not be considered uncertain or unclear, but construed according to its common meaning in the field. When referring to a trade name, it is intended to refer to its corresponding commercial product or its active ingredient.

Unless otherwise stated, the doses and ranges provided herein for anlotinib or the salt thereof are based on the molecular weight of the free base form for anlotinib.

As used herein, the term "pharmaceutical combination" refers to a combination of two or more active ingredients (administered as the respective active ingredients themselves, or as their respective derivatives like pharmaceutically acceptable salts or esters, prodrugs, or compositions) that are administered simultaneously or sequentially. The active substances can be administered to a subject simultaneously or sequentially in any order as a single formulation.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include an Fab fragment, an Fab' fragment, an F(ab')₂ fragment, an Fv fragment, an isolated CDR region, a single chain Fv molecule (scFv), an Fd fragment and other antibody fragments known in the art. The antibody and the fragment thereof may also include a recombinant polypeptide, a fusion protein, and a bispecific antibody. The anti-PD-L1 antibody and the fragment thereof disclosed herein can be of IgG1, IgG2, IgG3, or IgG4 isotype. The term "isotype" refers to the class of antibodies encoded by the heavy chain constant region gene. In one embodiment, the anti-PD-L1 antibody and the fragment thereof disclosed herein are of the IgG1 or IgG4 isotype. The anti-PD-L1 antibody and the fragment thereof of the present application can be derived from any species including, but not limited to, mouse, rat, rabbit, primate, llama, and human. The anti-PD-L1 antibody and the fragment thereof can be a chimeric antibody, a humanized antibody or an intact human antibody. In one embodiment, the anti-PD-L1 antibody is an antibody produced by a hybridoma cell line derived from a mouse. Thus, in one embodiment, the anti-PD-L1 antibody is a murine antibody. In another embodiment, the anti-PD-L1 antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The term "humanized antibody" refers to an antibody comprising complementarity determining regions (CDRs) derived from a non-human antibody, and framework and constant regions derived from a human antibody. For example, an anti-PD-L1 antibody disclosed herein may comprise CDRs derived from one or more murine antibodies as well as human framework and constant regions. Thus, in one embodiment, the humanized antibody disclosed herein binds to the same epitope on PD-L1 as the murine antibody from which the CDRs of the humanized antibody are derived. Exemplary humanized antibodies are disclosed herein. Additional anti-PD-L1 antibodies or variants thereof comprising the heavy and light chain CDRs disclosed herein can be generated using any human framework sequences, and are also included in the present application. In one embodiment, framework sequences suitable for use in the present application include those similar in structure to the framework sequences disclosed herein. Additional modifications may be made in the framework regions to improve the properties of the antibodies disclosed herein. Such additional framework modifications may include: chemical modifications, point mutations for reducing immunogenicity or removing T cell epitopes, or modifications reverting the mutations to residues in original germline sequences. In some embodiments, such modifications include those corresponding to the mutations exemplified herein, including reversions to germline sequences. For example, in one embodiment, one or more amino acids in the human VH and/or VL framework regions of the humanized antibodies disclosed herein are reverted to the corresponding amino acids in the parent murine antibodies. For example, for the VH and VL of humanized 5G11 and humanized 13C5 antibodies, several sites of framework amino acids of the template human antibodies described above are reverted to the corresponding amino acid sequences in the mouse 5G11 and 13C5 antibodies. In one embodiment, the amino acids at positions 53, 60 and/or 67 of the light chain variable region are reverted to the corresponding amino acids found at the positions in mouse 5G11 or 13C5 light chain variable region. In another embodiment, the amino acids at positions 24, 28, 30, 49, 73, 83 and/or 94 of the heavy chain variable region are reverted to the corresponding amino acids found at the positions in mouse 5G11 or 13C5 heavy chain variable region. In one embodiment, the humanized 5G11 antibody comprises: a light chain variable region, wherein the amino acid at position 60 is mutated from Ser (S) to Asp (D) and the amino acid at position 67 is mutated from Ser (S) to Tyr (Y); and a heavy chain variable region, wherein the amino acid at position 24 is mutated from Phe (F) to Val (V), the amino acid at position 49 is mutated from Ala (A) to Gly (G), the amino acid at position 73 is mutated from Thr (T) to Asn (N), and the amino acid at position 83 is mutated from Thr (T) to Asn (N). In one embodiment, the humanized 13C5 antibody comprises: a light chain variable region, wherein the amino acid at position 53 is mutated from Tyr (Y) to Lys (K); and a heavy chain variable region, wherein the amino acid at position 28 is mutated from Thr (T) to Ile (I), the amino acid at position 30 is mutated from Ser (S) to Arg (R), the amino acid at position 49 is mutated from Ser (S) to Ala (A), and the amino acid at position 94 is mutated from Tyr (Y) to Asp (D). Additional or alternative reverse mutations can be made in the framework regions of the humanized antibodies disclosed herein to improve the properties of the antibodies. The present application also includes humanized antibodies that bind to PD-L1 and comprise framework modifications corresponding to the exemplary modifications disclosed herein relative to any suitable framework sequence, as well as other framework modifications that otherwise improve antibody properties.

The present application provides an isolated antibody or a fragment thereof that binds to PD-L1, wherein the antibody can be produced by a hybridoma selected from the group consisting of the hybridomas designated herein as 13C5 and 5G11. Accordingly, the present application also includes hybridomas 13C5 and 5G11, and any hybridomas that produce the antibodies disclosed herein. The present application also provides isolated polynucleotides encoding the antibodies and the fragments thereof disclosed herein. The present application also includes expression vectors comprising the isolated polynucleotides, and host cells comprising the expression vectors.

The "isolated antibody" refers to an antibody that is substantially free of other antibodies having different antigenic specificities (e.g., an isolated antibody that specifically binds to PD-1 is substantially free of antibodies that specifically bind to antigens apart from PD-1). However, an isolated antibody that specifically binds to PD-1 may have cross-reactivity with other antigens (such as PD-1 molecules from different species). Furthermore, the isolated antibody may be substantially free of other cellular materials and/or chemicals.

The term "monoclonal antibody" ("mAb") refers to a non-naturally occurring preparation of antibody molecules of an individual molecule component (i.e., antibody molecules whose base sequences are substantially identical and which exhibit a single binding specificity and affinity for a particular epitope). mAb is an example of the isolated antibody. mAbs can be produced by hybridoma techniques, recombinant techniques, transgenic techniques, or other techniques known to those skilled in the art.

The antibody or the antigen-binding fragment thereof disclosed herein is specific for PD-L1. In one embodiment, the antibody or the fragment thereof is specific for PD-L1. In one embodiment, the antibody or the fragment thereof disclosed herein binds to human or primate PD-L1, but does not bind to PD-L1 from any other mammals. In another embodiment, the antibody or the fragment thereof does not bind to mouse PD-L1. The terms "human PD-L1", "hPD-L1" and "huPD-L1" and the like are used interchangeably herein and refer to human PD-L1 and variants or isotypes of human PD-L1. The terms "specific", "specificity" and "specifically" refer to that the antibody or fragment thereof binds to PD-L1 with greater affinity than any other targets.

The terms "treat", "treating" and "treatment" usually refer to acquiring needed pharmacological effect and/or physiological effect. In terms of partially or fully stabilizing or curing the disease and/or a side effect of the disease, the effect can be therapeutic. As used herein, "treat", "treating" and "treatment" encompass any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound disclosed herein for (i) treating a specific disease, condition or disorder; (ii) alleviating, ameliorating or eliminating one or more symptoms of a specific disease, condition or disorder, or (iii) preventing or delaying onset of one or more symptoms of the specific disease, condition or disorder described herein. The amount of active substance (e.g., the antibody or compound disclosed herein) constituting the "therapeutically effective amount" may vary according to factors such as the disease state, age, sex, and weight of the individual, and the ability of a therapeutic agent or a combination of therapeutic agents to elicit a desired response in the individual. The effective amount may also be determined routinely by those skilled in the art in accordance with their knowledge and the present disclosure.

The terms "administer", "administration" and "administering" refer to physically introducing the composition comprising a therapeutic agent to an entity using any of a variety of methods and delivery systems known to those skilled in the art. Routes of administration of immune checkpoint inhibitors (e.g., an anti-PD-1 antibody or an anti-PD-L1 antibody) include intravenous, intramuscular, subcutaneous, intraperitoneal, spinal, or other parenteral routes of administration, for example, by injection or infusion. As used herein, the phrase "parenteral administration" refers to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In some embodiments, the immune checkpoint inhibitor (e.g., an anti-PD-1 antibody or an anti-PD-L1 antibody) is administered by a non-parenteral route, and in some embodiments, by oral administration. Other non-parenteral routes include local, epidermal or mucosal routes, for example, intranasal, vaginal, rectal, sublingual or local routes. Administration may also be performed, e.g., once, multiple times, and/or over one or more extended periods of time.

The term "dose" refers to a dose administered to a patient without considering the weight or the body surface area (BSA) of the patient. For example, a 60 kg human and a 100 kg human will receive the same dose of antibody (e.g., 240 mg of anti-PD-1 antibody).

The term "pharmaceutically acceptable" is used herein for those compounds, materials, compositions, and/or dosage forms which are suitable for use in contact with the tissues of human beings and animals without excessive toxicity, irritation, allergic response, or other problems or complications.

The term "pharmaceutically acceptable salt" includes salts formed from a free base and an acid and salts formed from an acid and a free base, for example, hydrochloride, hydrobromide, nitrate, sulfate, phosphate, formate, acetate, trifluoroacetate, fumarate, oxalate, maleate, citrate, succinate, mesylate, benzenesulfonate and *p*-methylbenzenesulfonate, preferably, hydrochloride, hydrobromide, sulfate, formate, acetate, trifluoroacetate, fumarate, maleate, mesylate, *p*-methylbenzenesulfonate, sodium salt, potassium salt, ammonium salt and amino acid salt and so on. In the present application, in forming a pharmaceutically acceptable salt, the acid and the free base are in a molar ratio of about 1:0.2 to 1:5, preferably 1:0.5, 1:1, 1:2, 1:3, 1:4, 1:5, 1:6, 1:7 or 1:8.

As used herein, the terms "subject" and "patient" are used interchangeably. In some embodiments, the term "subject" or "patient" refers to a mammal. In some embodiments, the subject or patient is a mouse. In some embodiments, the subject or patient is a human.

The term "about" shall be understood to include a range of three standard deviations from a mean value or a standard tolerance range in a specific field. In some embodiments, the term "about" shall be understood as a variation not exceeding 0.5. The term "about" modifies all listed values thereafter. For example, "about 1, 2 and 3" represents "about 1", "about 2" and "about 3".

The term "single dose" refers to the smallest unit of packaging containing a certain quantity of pharmaceutical product; for example, in a box of seven capsules, each capsule is a single dose; or a vial of injection is a single dose. As used herein, the terms "single dose" and "unit dose" have the same meaning and are used interchangeably.

The term "multiple dose" consists of a plurality of single doses.

The term "pharmaceutical composition" refers to a mixture consisting of one or more of the active ingredients or pharmaceutical combinations thereof of the present application and a pharmaceutically acceptable excipient. The pharmaceutical composition is intended to facilitate the administration of the compound or the pharmaceutical combination thereof disclosed herein to a subject.

Unless otherwise stated herein, singular terms encompass plural forms, and vice versa.

As used herein, unless otherwise stated, the terms "comprise", "comprises" and "comprising" or equivalents thereof are open-ended statements and mean that elements, components and steps that are not specified may be included in addition to those listed.

Low-risk, medium-risk and high-risk patients are risk-stratified herein according to the criteria of the International Metastatic Renal Cell Carcinoma Database Consortium (IMDC). The criteria are specifically as follows:

| Risk factors | |
|---|---|
| 1 | < 1 year from time of diagnosis of primary kidney cancer to systemic therapy |
| 2 | Karnofsky Performance Status score < 80 |
| 3 | Corrected serum calcium^{a} > upper limit of normal |
| 4 | Hemoglobin < lower limit of normal |
| 5 | Neutrophils > upper limit of normal |
| 6 | Platelets > upper limit of normal |

### Prognostic risk stratification

| | |
|---|---|
| Low risk | 0 risk factor |
| Medium risk | 1-2 risk factors |
| High risk | 3-6 risk factors |

| | |
|---|---|
| Note: a. calculation formula for corrected serum calcium: corrected blood [Ca²⁺] (mg/L) = measured [Ca²⁺] (mg/L) + 0.8 × (4 - albumin value) | |

All patents, patent applications and other publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### DETAILED DESCRIPTION

For clarity, the present application is further described with the following examples, which are, however, not intended to limit the scope of the present application. All reagents used herein are commercially available and can be used without further purification. In the examples, the anti-PD-L1 antibody was prepared as described in WO2016022630, and after affinity chromatography, an eluate containing the antibody was obtained by conventional antibody purification methods.

### Example 1. Clinical Trial of Kidney Cancer

Kidney cancer subjects that met the criteria were given anti-PD-L1 antibody injections and anlotinib hydrochloride capsules. Response evaluation was performed once every 6 weeks (42 days). Subjects with disease control and tolerable adverse effects could be continuously medicated until clinical benefits were lost, until the toxicity was intolerable, until PD was given by response evaluation, or until the investigator deemed further medication inappropriate, whichever occurred first. Then the study ended.

### 1.1 Primary inclusion criteria:

1) Histopathologically confirmed clear cell renal cell carcinoma, including advanced renal cell carcinoma dominated by clear cell components;
2) Have not been subjected to systemic medication for locally advanced/metastatic diseases (those who failed previous cytokine therapy/were drug resistant were enrolled);
3) Non-surgically resectable locally advanced/metastatic diseases, and having at least one measurable lesion according to the Response Evaluation Criteria in Solid Tumors (RECIST 1.1); and
4) Age: 18-80 years old (calculated based on the date of signing the consent form); male or female; ECOG score: 0-1; expected survival ≥ 3 months.

### 1.2 Test drug

Anti-PD-L1 antibody hu5G 11-hIgG 1 injection: 1200 mg of the anti-PD-L1 antibody injection was diluted to 250 mL with normal saline, and the infusion time was 60 ± 10 min (from the beginning of the infusion of the anti-PD-L1 antibody injection until the end of the infusion of the anti-PD-L1 antibody injection and the completion of flushing the tube with normal saline (20 mL recommended)). The anti-PD-L1 antibody injection was administered on the first day, and once every 21 days-that is, 21 days were counted as a treatment cycle. (i.e., anti-PD-L1 antibody injection: 1200 mg, d1/q3w) strength: 600 mg/20 mL.

Anlotinib hydrochloride capsule (anlotinib dihydrochloride as active ingredient): once daily (orally taken before breakfast), 1 capsule (10 mg) each time. The oral administration was performed for 2 consecutive weeks and interrupted for 1 week-that is, 21 days were counted as a treatment cycle, and the drug was administered on days 1-14 of each cycle. Except in special circumstances, it was recommended to take it at a fixed time every day. (i.e., anlotinib hydrochloride capsule: 10 mg/qd, d1-14/q3w)

The investigator could adjust the dose of the anlotinib hydrochloride capsule, e.g., to 12 mg, 10 mg or 8 mg, according to the disease condition, safety and other aspects.

### 1.3 Evaluation criteria

Safety evaluation: the NCI-CTCAE 5.0 criteria were adopted.

Response evaluation: the RECIST 1.1 criteria and the iRECIST criteria were adopted.

### 1.4 Endpoints

### Primary endpoint

Effectiveness indicator: progression-free survival (PFS) given by the Independent Review Committee's (IRC) evaluation.

### Secondary endpoints

Effectiveness indicators: progression-free survival (PFS), overall survival (OS), objective response rate (ORR = complete response (CR) + partial response (PR)), disease control rate (DCR = CR + PR + stable disease (SD)), duration of response (DOR), 12-month progression-free survival rate, 12-month and 24-month overall survival rates, and quality of life score, given by the investigator's evaluation; and immune-related effectiveness indicators (such as iPFS).

Safety indicators: incidence and severity of adverse events (AEs), laboratory test value abnormalities and serious adverse events (SAEs).

Biomarker: the correlation of PD-L1 expression with response.

Immunogenicity of anti-PD-L1 antibody injection: anti-drug antibody (ADA) positive rate, and so on.

### 1.5 Response

Preliminary studies showed that subjects could benefit from the treatment and achieve significant response. Up to the day the data were acquired, in this study of using anlotinib hydrochloride in combination with the anti-PD-L1 antibody to treat renal cell carcinoma (anlotinib hydrochloride capsules were administered to the subjects at a dose of 12 mg/qd, d1-14/q3w), 34 subjects in the test group (anlotinib hydrochloride combined with anti-PD-L1 antibody group) had completed at least 2 cycles of treatment, and some of them had completed 10 cycles of treatment; according to the statistics by best response, 16 patients (16/34) had achieved stable disease, and 17 patients (17/34) had achieved partial response, as detailed in Table 1, in which the patients were classified according to the IMDC criteria into low-risk patients, medium-risk patients, and high-risk patients.

**Table 1. Summary of best response after completion of at least 2 cycles of treatment (N = 34)**

| Response | Number of subjects (ratio) | | | |
|---|---|---|---|---|
| | Low-risk patients | Medium-risk patients | High-risk patients | Total of patients |
| CR | 0/12 (0.00%) | 0/16 (0.00%) | 0/6 (0.00%) | 0/34 (0.00%) |
| PR | 7/12 (58.33%) | 8/16 (50.00%) | 2/6 (33.33%) | 17/34 (50.00%) |
| SD | 5/12 (41.67%) | 8/16 (50.00%) | 3/6 (50.00%) | 16/34 (47.06%) |
| PD | 0/12 (0.00%) | 0/16 (0.00%) | 176 (16.67%) | 1 */34 (2.94%) |
| ORR | 7/12 (58.33%) | 8/16 (50.00%) | 2/6 (33.33%) | 17/34 (50.00%) |
| DCR | 12/12(100.00%) | 16/16 (100.00%) | 5/6 (83.33%) | 33/34 (97.06%) |

| | | | | |
|---|---|---|---|---|
| *: The response evaluated as iUPD (the iRECIST criteria), further response evaluation was required. | | | | |

In addition, the results show that the combination treatment regimen produced a quick response in the treatment of kidney cancer; as shown in Table 2, after 2 cycles of treatment, the results of response evaluation showed that in the test group, 25 patients (25/34) had stable disease and 8 patients (8/34) had partial response: the objective response rate (ORR) was up to 23.53% and the disease control rate (DCR) up to 97.06%.

**Table 2. Summary of response in first tumor evaluation (2 cycles of treatment completed) (N = 34)**

| Response | Number of subjects (ratio) | | | |
|---|---|---|---|---|
| | Low-risk patients | Medium-risk patients | High-risk patients | Total of patients |
| CR | 0/12 (0.00%) | 0/16 (0.00%) | 0/6 (0.00%) | 0/34 (0.00%) |
| PR | 2/12 (16.67%) | 6/16 (37.50%) | 0/6 (0.00%) | 8/34 (23.53%) |
| SD | 10/12 (83.33%) | 10/16 (62.50%) | 5/6 (83.33%) | 25/34 (73.53%) |
| PD | 0/12 (0.00%) | 0/16 (0.00%) | 176 (16.67%) | 1*/34 (2.94%) |
| ORR | 2/12 (16.67%) | 6/16 (37.50%) | 0/6 (0.00%) | 8/34 (23.53%) |
| DCR | 12/12(100.00%) | 16/16 (100.00%) | 5/6 (83.33%) | 33/34 (97.06%) |

| | | | | |
|---|---|---|---|---|
| ^{∗}: The response evaluated as iUPD (the iRECIST criteria), further response evaluation was required. | | | | |

## Claims

1. A pharmaceutical combination or pharmaceutical composition for use in treating kidney cancer, comprising: an anti-PD-L1 antibody, and anlotinib or a pharmaceutically acceptable salt thereof.

2. The pharmaceutical combination or pharmaceutical composition according to claim 1, wherein the pharmaceutical combination or pharmaceutical composition comprises: a pharmaceutical composition of the anti-PD-L1 antibody, and a pharmaceutical composition of anlotinib or the pharmaceutically acceptable salt thereof.

3. The pharmaceutical combination or pharmaceutical composition according to claim 1 or 2, packaged in a kit further comprising instructions for combined use of the PD-L1 antibody and anlotinib or the pharmaceutically acceptable salt thereof in treating kidney cancer.

4. The combined pharmaceutical composition according to any one of claims 1 to 3, wherein the combined pharmaceutical composition comprises the anti-PD-L1 antibody and anlotinib in a weight ratio of (0.35-29):1, preferably (3.5-29):1, more preferably (3.5-14.5):1, and most preferably (7-14.5):1.

5. The combined pharmaceutical composition according to any one of claims 1 to 4, wherein the combined pharmaceutical composition is a formulation suitable for administration within a single treatment cycle (e.g., a 21-day treatment cycle) and comprises a pharmaceutical composition comprising 600-2400 mg of the anti-PD-L1 antibody and a pharmaceutical composition comprising 84-168 mg of anlotinib.

6. Use of the pharmaceutical combination or pharmaceutical composition according to any one of claims 1 to 5 for preparing a medicament for treating kidney cancer.

7. Use of a pharmaceutical combination or pharmaceutical composition comprising an anti-PD-L1 antibody and anlotinib or a pharmaceutically acceptable salt thereof for preparing a medicament for treating kidney cancer.

8. Use of a pharmaceutical combination or pharmaceutical composition comprising an anti-PD-L1 antibody and anlotinib or a pharmaceutically acceptable salt thereof for treating kidney cancer.

9. The use according to any one of claims 6 to 8, wherein the anti-PD-L1 antibody and anlotinib or the pharmaceutically acceptable salt thereof are in a weight ratio of (0.35-29):1, (3.5-29):1, (3.5-14.5):1 or (7-14.5):1.

10. The use according to any one of claims 6 to 9, comprising: a pharmaceutical composition comprising 600-2400 mg of the anti-PD-L1 antibody and a pharmaceutical composition comprising 6 mg, 8 mg, 10 mg and/or 12 mg of anlotinib or the pharmaceutically acceptable salt thereof in a unit dose.

11. The use according to claim 10, comprising: a pharmaceutical composition comprising 600-2400 mg of the anti-PD-L1 antibody provided in multiple-dose form and a pharmaceutical composition comprising 6 mg, 8 mg, 10 mg and/or 12 mg of anlotinib or the pharmaceutically acceptable salt thereof in a unit dose.

12. The use according to any one of claims 6 to 11, wherein the anti-PD-L1 antibody is administered once a week, once every 2 weeks, once every 3 weeks, or once every 4 weeks.

13. The use according to any one of claims 6 to 12, wherein one treatment cycle comprises 21 days; the PD-L1 antibody is administered on the first day of each treatment cycle, and anlotinib or the pharmaceutically acceptable salt thereof is administered daily on days 1-14 of each cycle.

14. The use according to claim 13, wherein 1200 mg of the PD-L1 antibody is administered on the first day of each treatment cycle, and 6 mg, 8 mg, 10 mg and/or 12 mg of anlotinib or the pharmaceutically acceptable salt thereof is administered daily on days 1-14 of each treatment cycle.

15. The use according to any one of claims 6 to 14, wherein the pharmaceutical composition of the anti-PD-L1 antibody is a solution for injection; the pharmaceutical composition of anlotinib or the pharmaceutically acceptable salt thereof is an oral solid formulation.

16. The pharmaceutical combination or pharmaceutical composition according to any one of claims 1 to 5, or the use according to any one of claims 6 to 15, wherein the kidney cancer is advanced kidney cancer.

17. The pharmaceutical combination or pharmaceutical composition according to any one of claims 1 to 5, or the use according to any one of claims 6 to 15, wherein the kidney cancer is clear cell renal cell carcinoma or non-clear cell renal cell carcinoma.

18. The pharmaceutical combination or pharmaceutical composition according to any one of claims 1 to 5, or the use according to any one of claims 6 to 15, wherein the kidney cancer is clear cell renal cell carcinoma, or a renal cell carcinoma dominated by clear cell components.

19. The pharmaceutical combination or pharmaceutical composition according to any one of claims 1 to 5, or the use according to any one of claims 6 to 15, wherein a patient with the kidney cancer is a low-risk kidney cancer patient, or a medium-risk kidney cancer patient, or a high-risk kidney cancer patient.

20. The pharmaceutical combination or pharmaceutical composition according to any one of claims 1 to 5, or the use according to any one of claims 6 to 15, wherein the kidney cancer is treatment-naive renal cell carcinoma.

21. The pharmaceutical combination or pharmaceutical composition according to any one of claims 1 to 5, or the use according to any one of claims 6 to 15, wherein the kidney cancer is treatment-naive advanced renal cell carcinoma.

22. The pharmaceutical combination or pharmaceutical composition according to any one of claims 1 to 5, or the use according to any one of claims 6 to 15, wherein the kidney cancer is a kidney cancer that has not been subjected to systemic prior treatment.

23. The pharmaceutical combination or pharmaceutical composition or the use according to claim 22, wherein the prior treatment includes surgical treatment, radiotherapy, or medication.

24. The pharmaceutical combination or pharmaceutical composition or the use according to claim 23, wherein the medication includes administration of one or more of cyclophosphamide, ifosfamide, vincristine, prednisone, prednisolone, methylprednisolone, fluorouracils (including 5-fluorouracil, tegafur-gimeracil-oteracil potassium, tegafur, and capecitabine), bicins (including adriamycin, pirarubicin, amrubicin, epirubicin, aclarubicin, and idarubicin), antifolates (including methotrexate), cytarabine, azacitidine, fludarabine, bendamustine, mitoxantrone, etoposide, procarbazine, gemcitabine, mesna, dexamethasone, leucovorin, platins (including oxaliplatin, cisplatin, carboplatin, nedaplatin, dicycloplatin, and miriplatin), camptothecin analogs (including camptothecin, hydroxycamptothecin, irinotecan, and topotecan), taxanes (including paclitaxel, albumin-bound paclitaxel and docetaxel), IL-2, INF-α, sunitinib, sorafenib, pazopanib, axitinib, lenvatinib, cabozantinib, everolimus, temsirolimus, tivozanib, bevacizumab, pembrolizumab, ipilimumab, nivolumab and avelumab.

25. The pharmaceutical combination or pharmaceutical composition according to any one of claims 1 to 5, or the use according to any one of claims 6 to 15, wherein the anti-PD-L1 antibody and anlotinib or the pharmaceutically acceptable salt thereof are each in the form of a pharmaceutical composition and are administered simultaneously, sequentially or at intervals.

26. A kit for use in treating kidney cancer, comprising: an anti-PD-L1 antibody, and anlotinib or a pharmaceutically acceptable salt thereof, wherein the anti-PD-L1 antibody is contained in a first compartment, and anlotinib or the pharmaceutically acceptable salt thereof is contained in a second compartment.

27. The kit according to claim 26, wherein the kit is suitable for administration within a single treatment cycle and comprises a pharmaceutical composition comprising 600-2400 mg of the anti-PD-L1 antibody and a pharmaceutical composition comprising 84-168 mg of anlotinib.

28. The pharmaceutical combination or pharmaceutical composition according to any one of claims 1 to 5, the use according to any one of claims 6 to 15, or the kit according to claim 26 or 27, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

29. The pharmaceutical combination or pharmaceutical composition according to any one of claims 1 to 5, the use according to any one of claims 6 to 15, or the kit according to claim 26 or 27, wherein the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

30. The pharmaceutical combination or pharmaceutical composition according to any one of claims 1 to 5, the use according to any one of claims 6 to 15, or the kit according to claim 26 or 27, wherein the anti-PD-L1 antibody comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

31. The pharmaceutical combination or pharmaceutical composition according to any one of claims 1 to 5, the use according to any one of claims 6 to 15, or the kit according to claim 26 or 27, wherein the anti-PD-L1 antibody comprises the following amino acid sequences: a heavy chain variable region having at least 80% homology to an amino acid sequence set forth in SEQ ID NO: 13 or SEQ ID NO: 14; and a light chain variable region having at least 80% homology to an amino acid sequence set forth in SEQ ID NO: 15 or SEQ ID NO: 16.

32. The pharmaceutical combination or pharmaceutical composition according to any one of claims 1 to 5, the use according to any one of claims 6 to 15, or the kit according to claim 26 or 27, wherein the anti-PD-L1 antibody comprises: a heavy chain variable region selected from the group consisting of heavy chain variable regions of hu13C5-hIgG1, hu13C5-hIgG4, hu5G11-hIgG1 and hu5G11-hIgG4 humanized antibodies; and a light chain variable region selected from the group consisting of light chain variable regions of hu 13 C5 -hIgG 1, hu13C5-hIgG4, hu5G11-hIgG1 and hu5G11-hIgG4 humanized antibodies.

33. A method for treating kidney cancer, comprising administering to a patient in need an effective amount of a pharmaceutical combination or pharmaceutical composition comprising an anti-PD-L1 antibody and anlotinib or a pharmaceutically acceptable salt thereof.
